# NEUE EUROPÄISCHE PATENTSCHRIFT

(11) **EP 1 577 389 B2**
(45) Veröffentlichungstag und Bekanntmachung des Hinweises auf die Entscheidung über den Einspruch: **16.08.2017**
(45) Hinweis auf die Patenterteilung: 25.07.2007
(21) Anmeldenummer: 05004214.2
(22) Anmeldetag: 06.06.1996
(51) Int. Cl.: C12N 15/11

(54) **Verfahren zur enzymatischen Reaktion an Nukleinsäuren**
A method for carrying out an enzymatic reaction on nucleic acids
Procédé pour la réaction enzymatique sur des acides nucléiques

(30) Priorität: 08.06.1995 DE 19520398; 12.10.1995 DE 19537985
(43) Veröffentlichungstag der Anmeldung: 21.09.2005
(62) Teilanmeldung aus: 02016299.6
(73) Patentinhaber: Roche Diagnostics GmbH, 68305 Mannheim (DE)
(72) Erfinder: Kleiber, Jörg, 82377 Penzberg (DE); Walter, Thomas, 82377 Penzberg (DE); Harttig, Herbert, 67434 Neustadt (DE); Menning, Martin, 66287 Quierschied (DE); Lesniak, Christoph, 87474 Buchenberg (DE); Riedling, Michael, 82377 Penzberg (DE); Schmidt, Helmut K., 66130 Saarbrücken-Güdingen (DE)
(74) Vertreter: Teschemacher, Andrea

(56) Entgegenhaltungen:
- EP-A- 0 389 063
- EP-A2- 0 343 934
- WO-A-91/12079
- WO-A-95/04140
- WO-A1-93/10162
- WO-A1-95/06652
- DE-A1- 3 014 036
- DE-A1- 4 307 262
- DE-A1- 4 307 262
- DE-T2- 3 850 273
- US-A- 4 233 169
- ALDERTON R.P. ET AL: 'Magnetic bead purification of M13 DNA sequencing templates' ANALYTICAL BIOCHEMISTRY 201 1992, Seiten 166 - 169
- Vergleichsversuche von der Patentinhaberin (eingereicht am 08.12.2008)
- ARNHEIM K. ET AL: 'Herder Lexikon der Biochemie und Molekular-biologie', 1995, SPEKTRUM AKADEMISCHER VERLAG GMBH, HEIDELBERG Seite 25
- Erklärung von Herrn Michael Riedling
- Erklärung Thomas Walter
- BOOM R. ET AL: 'Rapid and simple method for purification of nucleic acids' JOURNAL OF CLINICAL MICROBIOLOGY Bd. 28, Nr. 3, März 1990, Seiten 495 - 503
- HENGEN P.N.: 'Mini-prep plasmid DNA isolation and purification using silica-based resins' MOLECULAR BIOLOGY 1995,
- STACY-PHIPPS S. ET AL: 'Multiplex PCR assay and simple preparation method for stool specimens detect enterotoxigenic Escherichia coli DNA during course of infection' JOURNAL OF CLINICAL MICROBIOLOGY Bd. 33, Nr. 5, Mai 1995, Seiten 1054 - 1055
- ATTAL J. ET AL: 'A simple method of DNA extraction from whole tissue and blood using glass powder for detection of transgenic animals by PCR' TRANSGENIC RESEARCH Bd. 4, 1995, Seiten 149 - 150
- KAUR R. ET AL: 'Selective recovery of DNA fragments from silica particles: effect of A-T content and elution conditions' NUCLEIC ACIDS RESEARCH Bd. 23, Nr. 23, 1995,
- 'An inexpensive alternative to glassmilk for DNA purification' TIG Bd. 11, Nr. 1, Januar 1995,
- 'Methods and reagents' ELSEVIER SCIENCE April 1994, Seite 182
- 'Methods and reagents: recovering DNA from agarose gels' TIBS 19 September 1994, Seite 388
- CARTER M.J. ET AL: 'An inexpensive and simple method for DNA purifications on silica particles' NUCLEIC ACIDS RESEARCH Bd. 21, Nr. 4, 1993,
- BUFFONE G.J. ET AL: 'Improved amplification of cytomegalovirus DNA from urine after purification of DNA with glass beads' CLIN. CHEM. Bd. 37, Nr. 11, 1991, Seiten 1945 - 1949
- BOOM R. ET AL: 'Rapid purification of Hepatitis B virus DNA from serum' JOURNAL OF CLINICAL MICROBIOLOGY Bd. 29, Nr. 9, September 1991, Seite 1804
- SCHUURMAN T. ET AL: 'Reduced PCR sensitivity due to impaired DNA recovery with the magNA pure LC total nucleic acid isolation kit' JOURNAL OF CLINICAL MICROBIOLOGY Bd. 43, Nr. 9, September 2005, Seiten 4616 - 4617
- Annex 2- Zusammenfassende Ausführungen zu Vergleichsversuchen
- D16a/ Molecular Biology: Current Innovations and Future Trends part 1
- CV T. Walter
- Adsorption aus Wikipedia & Van-der-Waals-Kräfte aus Wikipedia

## Beschreibung

Gegenstand der Erfindung ist ein Verfahren zur Isolierung von und enzymatischen Reaktion an Nukleinsäuren, umfassend Inkontaktbringen einer Probe, welche die Nukleinsäuren in einer Flüssigkeit enthält, mit magnetischen Partikeln mit Glasoberflächen unter Bedingungen, bei denen die Nukleinsäuren in nativer Form an die Glasoberfläche binden können, Binden der Nukleinsäuren in nativer Form durch Adsorption an die Glasoberfläche der magnetischen Partikel , Abtrennen der gebundenen Nukleinsäuren von der Flüssigkeit und Einsetzen der Nukleinsäuren als Substrat in einer enzymatischen Reaktion.

Manche biologische Materialien, insbesondere Nukleinsäuren, stellen im Hinblick auf ihre Isolierung aus der natürlichen Umgebung besondere Anforderungen. Zum einen sind sie oft in sehr geringen Konzentrationen vorhanden und zum anderen befinden sie sich oft in Nachbarschaft vieler anderer fester und gelöster Substanzen, die ihre Isolierung, beziehungsweise Bestimmung, beeinträchtigen.

In jüngerer Zeit hat es daher nicht an Versuchen gefehlt, Verfahren und Materialien zur Isolierung von Nukleinsäuren aus ihrer natürlichen Umgebung vorzuschlagen. Aus Proc. Natl. Acad. USA 76, 615-619 (1979) ist die Bindung von Nukleinsäuren aus Agarosegelen in Gegenwart von Natriumjodid in gemahlenem Flintglas beschrieben.

In Anal. Biochem. 121, 382 - 387 (1982) ist die Reinigung von Plasmid DNA aus Bakterien an Glasstaub in Gegenwart von Natriumperchlorat beschrieben.

In DE-A 37 34 442 ist die Isolierung von einzelsträngiger M13 Phagen-DNA an Glasfaserfiltern durch Ausfällung der Phagenpartikel mit Hilfe von Essigsäure und Lyse der Phagenpartikel mit Perchlorat beschrieben. Die an die Glasfaserfilter gebundenen Nukleinsäuren werden nach Waschen mit einem methanolhaltigen Puffer in Tris/EDTA-Puffer eluiert.

In Anal. Biochem. 175, 196- 201 (1988) ist ein ähnliches Verfahren zur Reinigung von DNA aus Lambdaphagen beschrieben.

In WO 95/04140 wird ein Verfahren zur Ampflifikation gereinigter Nukleinsauren aus einem Gram-positiven Organismus aus einer biologischen Probe beschrieben. Die biologische Probe wird hierbei mit Lysozym, einer chaotropen Substanz und einer festen Phase, z.B. Silikapartikeln, gemischt. Die Nukleinsäuren, die an die feste Phase binden, werden dann von der restlichen Probe separiert. Anschließend werden die Silikapartikel-Nukleinsäure-Komplexe mit Hilfe von Pufferlösungen gereinigt, die Nukleinsäuren gefällt, eluiert und amplifiziert.

Den bisher genannten Verfahren des Standes der Technik ist die selektive Bindung von Nukleinsäuren an Glasoberflächen in chaotropen Salzlösungen gemeinsam, wobei die Nukleinsäure von Verunreinigungen wie Agarose, Proteinen oder Zelltrümmern abgetrennt wird. Zur Separierung der Glaspartikel von den Verunreinigungen wird nach dem Stand der Technik entweder Zentrifugation von Partikeln oder Durchsaugen von Flüssigkeiten durch Glasfaserfilter verwendet. Hierbei handelt es sich jedoch um einen limitierenden Schritt, der die Verarbeitung großer Probenzahlen stark behindert.

In Anal. Biochem. 201, 166-169 (1992) bzw. PCT GB 91/00212 ist die Verwendung von Magnetpartikeln zur Immobilisierung von Nukleinsäuren nach Ausfällung durch Zugabe von Salz und Ethanol beschrieben. Hierbei findet eine Agglutination der Nukleinsäuren unter Einschluss der Magnetpartikel statt. Das Agglutinat wird von dem ursprünglichen Lösungsmittel durch Anlegen eines Magnetfeldes und Waschen getrennt. Nach einem Waschschritt werden die Nukleinsäuren in einem Trispuffer gelöst. Dieses Verfahren hat jedoch den Nachteil, dass die Ausfällung nicht selektiv für Nukleinsäuren ist, sondern eine Vielzahl von festen und gelösten Stoffe mitagglutiniert werden. So ist es durch dieses Verfahren nicht möglich, eventuell vorhandene Inhibitoren für bestimmte enzymatische Reaktionen in ausreichendem Masse zu entfernen.

In US-A-4,233,169 ist ein poröses Glas beschrieben, das Magnetpartikel eingelagert enthält.

Auf dem Markt befindet sich derzeit auch sogenanntes magnetisches, poröses Glas, welches Magnetpartikelchen in einer porösen, partikulären Glasmatrix enthält, und wobei die Oberfläche von einer streptavidinhaltigen Schicht überzogen ist. Dieses Produkt kann zur Isolierung von biologischen Materialien, zum Beispiel Proteinen oder Nukleinsäuren verwendet werden, wenn diese in einem aufwendigen Vorbereitungsschritt so modifiziert werden, dass diese kovalent an Biotin gebunden sind.

Aufgabe der Erfindung war es, ein einfaches und für die Routine-Diagnostik geeignetes Verfahren zur enzymatischen Reaktion an Nukleinsäuren bereitzustellen.

Gegenstand der Erfindung ist ein Verfahren zur Isolierung von und enzymatischen Reaktion an Nukleinsäuren, umfassend Inkontaktbringen einer Probe, welche die Nukleinsäuren in einer Flüssigkeit enthält, mit magnetischen Partikeln mit Glasoberflächen unter Bedingungen, bei denen die Nukleinsäuren in nativer Form an die Glasoberfläche binden können, Binden der Nukleinsäuren in nativer Form durch Adsorption an die Glasoberfläche der magnetischen Partikel, Abtrennen der gebundenen Nukleinsäuren von der Flüssigkeit und Einsetzen der Nukleinsäuren als Substrat in einer enzymatischen Reaktion.

Als Partikel bezeichnet der Fachmann feste Materialien mit einem geringen Durchmesser. Manchmal bezeichnet man solche Partikel auch als Pigmente. Im Sinne der vorliegenden Erfindung sind besonders Partikel geeignet, die eine durchschnittliche Korngröße von weniger als 100 µm haben. Besonders bevorzugt weisen sie eine durchschnittliche Komgröße von zwischen 10 und 60 µm auf. Bevorzugt ist die Korngrößenverteilung relativ homogen, insbesondere liegen nahezu keine Teilchen < 10 µm oder > 60 µm vor.

Als magnetisch werden Materialien bezeichnet, die durch einen Magnet angezogen werden können, d. h. beispielsweise ferromagnetische oder superparamagnetische Materialien. Als magnetisch werden auch Materialien verstanden, die als weich magnetische Materialien bezeichnet werden, z. B. Ferrite. Besonders bevorzugt im Sinne der Erfindung sind ferromagnetische Materialien, insbesondere wenn sie noch nicht vormagnetisiert wurden. Unter Vormagnetisierung ist in diesem Zusammenhang das Inkontaktbringen mit einem Magneten zu verstehen, wodurch die Remanenz erhöht wird. Besonders bevorzugt sind ferromagnetische Materialien, wie z. B. Magnetit (Fe₃O₄) oder Fe₂O₃.

Unter einer äußeren Oberfläche eines Partikels wird die zusammenhängende Oberfläche verstanden, von der in Richtung auf die Umgebung des Partikels Senkrechte gebildetwerden können, die dasselbe Partikel nicht noch einmal schneiden.

Unter eine Pore wird eine Ausnehmung in der äußeren Oberfläche des Partikels verstanden, bei denen die Oberfläche soweit in das Partikel hineinreicht, dass eine in der Ausnehmung auf der Oberfläche gebildete gedachte Senkrechte in Richtung auf die nächstliegende Umgebung des Partikels das Partikel mindestens 1 mal schneidet. Poren reichen außerdem tiefer als ein Radius der Pore in das Partikel hinein.

Unter einem Glas im Sinne der vorliegenden Erfindung wird ein siliciumhaltiges amorphes Material verstanden. Das Glas kann weitere Materialien enthaltenen, z. B.

| | |
|---|---|
| B₂O₃ | (0-30%), |
| Al₂O₃ | (0-20%), |
| CaO | (0-20%), |
| BaO | (0-10%), |
| K₂O | (0-20%), |
| N₂O | (0-20%), |
| MgO | (0-18%), |
| Pb₂O₃ | (0-15%). |

In geringerem Umfang von 0 - 5 % können auch eine Vielzahl anderer Oxide, wie z. B. Mn₂O₃ TiO₂, As₂O₃, Fe₂O₃, CuO, CoO usw. enthalten sein. Als besonders wirksam haben sich Oberflächen einer Zusammensetzung von Borsilikatglas, Flintglas oder Silica erwiesen. Unter dem Gesichtspunkt der Ausbeute an Nukleinsäuren besonders bevorzugte Borsilikatgläser haben einen Boroxidgehalt von mehr als 25 %; als besonders wertvoll wurde ein Glas der Zusammensetzung SiO₂/B₂O₃ 70/30 erkannt. Besonders bevorzugt im Sinne der Erfindung sind Gläser, die durch den sogenannten Gel-Solprozess und anschließendes Trocknen und Verdichten der gebildeten Schicht gebildet werden. Dieser Prozess ist in seinen Grundzügen bekannt und wurde z. B. in C.J. Brinker, G.W. Scherer "Sol Gel science - The physics and chemistry of Sol Gel Processing", Academic Press Inc. 1990 und Sol-Gel Optics, Processing and Applications LisaC. Klein Ed. KluwerAcademic Publishers 1994, Seite 450 ff. sowie in DE-A-1941191, DE-A-3719339, DE-A-4117041 und DE-A-4217432 beschrieben. Er wurde allerdings bisher noch nicht für magnetische Partikel beschrieben. Dass hiermit magnetische Partikel erzeugt werden können, die bei der Isolierung von Nukleinsäuren, ganz überraschende Eigenschaften haben, war nicht zu erwarten. Im Gel-Sol-Prozess werden Alkoxide von netzwerksbildenden Komponenten, z. B. SiO₂, B₂O₃, Al₂O₃, TiO₂, ZrO₂, GeO₂ zusammen mit Oxiden und Salzen anderer Komponenten, z. B. in alkoholischer Lösung, vorgelegt und hydrolysiert. In der Gleichung ist die Herstellung von einem Natriumboroaluminiumsilikatglas aufgeführt.

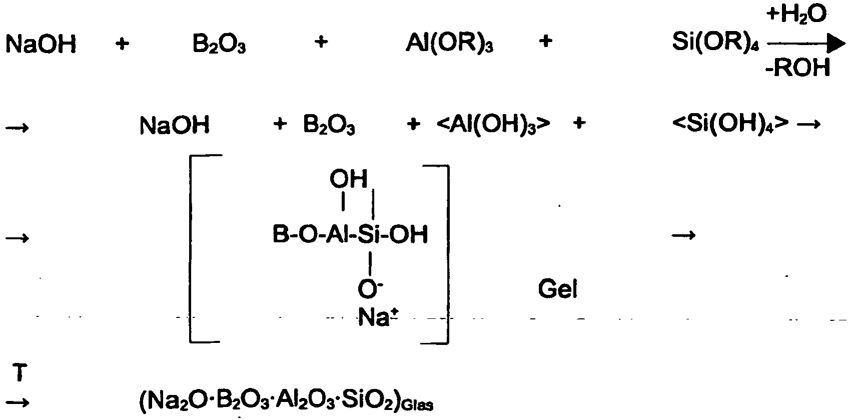

Durch die Zugabe von Wasser wird der Hydrolyseprozess der Ausgangskomponenten in Gang gesetzt. Die Reaktion verläuft relativ rasch, da die Alkaliionen katalytisch auf die Hydrolysegeschwindigkeit des Kieselsäureesters einwirken. Nach Ablauf der Gelbildung kann das entstehende Gel getrocknet und durch einen thermischen Prozess zu einem Glas verdichtet werden.

Das Mengenverhältnis Sol/Pigment hat einen erheblichen Einfluss auf die Ausbeute an magnetischem Pigment. Grenzen sind dadurch gegeben, dass der Pigmentanteil so gering ist, dass eine noch pump- und sprühfähige Masse entsteht. Bei zu geringem Pigmentanteil wird der Feinanteil, z. B. von nicht-magnetischem Material zu groß und stört. Als im Hinblick auf die Pigmentausbeute zweckmäßige Mengenverhältnisse wurden 10 bis 25 g Pigment/100 ml Sol gefunden.

Die Aufschlämmung wird zur Entstehung eines Pulvers bevorzugt durch eine Düse versprüht und das Aerosol auf einer Fallstrecke getrocknet. Die Düse wird bevorzugt geheizt, um die Trocknung der Aufschlämmung zu beschleunigen. Abhängig von der Geometrie der Düse beträgt die Düsentemperatur bevorzugtca. 120 bis 200 °C. Ein Kompromiss wird gefunden durch ausreichende Verdampfungsgeschwindigkeit, jedoch Vermeiden von Verspritzen.

Im Hinblick auf die Ausbeute ist die Verdichtungstemperatur möglichst hoch zu wählen. Ist sie jedoch zu hoch, verkleben die Partikel untereinander und es bilden sich Agglomerate, die herausgesiebt werden sollten. Die Nachbehandlung unter Luft führt bei zu hohen Temperaturen zu einem Verlust der magnetischen Eigenschaften, weshalb zu hohe Temperaturen vermieden werden sollten.

Unter einer im wesentlichen porenfreien Oberfläche wird eine Oberfläche verstanden, die zu weniger als 5 %, bevorzugt weniger als 2 %, besonders bevorzugt weniger als 0,1 %, mit Poren der oben stehenden Definition durchsetzt ist. Sollten Poren vorhanden sein, so haben diese bevorzugt einen Durchmesservonwenigerals 10, besonders bevorzugt 1 nm.

Besonders bevorzugt im Sinne der Erfindung werden Partikel verwendet, die einen Kern aus mit TiO₂ beschichteten Glimmer und darauf immobilisierten Magnetitpartikeln enthalten, wobei der so gebildete Verbundstoff von der Glasschicht umschlossen ist. Sowohl der Kern als auch die Magnetitpartikel sind kristallin und nicht porös. Die Räume auf der Oberfläche des Glimmers, welche nicht von den Magnetitpartikeln besetzt ist, sind von einer dickeren Glasschicht überzogen als die Spitzen der Magnetitpartikel, so dass sich eine im wesentlichen nicht-poröse Glasoberfläche ergibt.

Die Nichtporosität der magnetischen Partikel bezieht sich nur auf die äußere Oberfläche, nicht auf das Innere des Partikels, so dass das Partikel in seinem Inneren porös sein kann, wenn die Oberfläche nur von im wesentlichen porenfreiem Glas oder einer Glasoberfläche mit Poren eines Durchmessers von weniger als 10 nm umschlossen ist.

Überraschenderweise sind die magnetischen Partikel besonders vorteilhaft zur Isolierung von Nukleinsäuren aus Proben geeignet. Insbesondere werden lange Nukleinsäuren sehr wenig oder gar nicht zerstört, wenn sie daran immobilisiert werden. Das Material des Kerns ist darüber hinaus eine natürliche Ressource und somit ökologisch wenig bedenklich. Die Herstellung der Partikel ist darüber hinaus sehr wenig aufwendig und kostengünstig.

Im erfindungsgemäßen Verfahren werden bevorzugt ferromagnetische Partikel mit einer Glasoberfläche verwendet. Im Stand der Technik sind superparamagnetische Partikel beschrieben. Es hat sich nun herausgestellt, dass ferromagnetische Partikel, wenn sie mit einer Glasoberfläche überzogen sind, erhebliche Vorteile bei der Isolierung von Nukleinsäuren aufweisen. Solange die ferromagnetischen Partikel noch keinem Magnetfeld ausgesetzt waren, sedimentieren sie ausschließlich unter Einfluss der Schwerkraft. Sie sind durch Schütteln einfach und schnell wieder zu suspendieren. Der Vorgang der Abscheidung ohne Magnetfeldeinfluss verläuft dabei bevorzugt langsamer als die Immobilisierung von Nukleinsäuren an ihrer Oberfläche. Die ferromagnetischen Partikel können auf einfache Weise mittels eines Magneten an einer bestimmten Stelle der Probenflüssigkeit gesammelt werden, um die Flüssigkeit von den Partikeln und somit den immobilisierten Nukleinsäuren zu trennen.

Die Glasoberfläche der erfindungsgemäßen ferromagnetischen Partikel kann porenfrei sein oder aber Poren enthalten. Aus den oben genannten Gründen ist es bevorzugt, dass die äußere Oberfläche auch der ferromagnetischen Partikel im wesentlichen porenfrei ist oder Poren eines Durchmessers von weniger als 10 nm aufweist. Auch die ferromagnetischen Partikel haben bevorzugt eine Korngröße zwischen 10 und 60 µm, besonders bevorzugt 20 und 50 µm. Besonders bevorzugt sind Partikel, bei denen eventuell in der Oberfläche vorhandene Poren einen Durchmesser von weniger als 10, besonders bevorzugt 1 nm haben. Ein Beispiel für einen ferromagnetischen Partikel ist der oben genannte Verbundstoff aus Glimmer- und Magnetitpartikeln, umschlossen von einer Glasschicht.

Das erfindungsgemäße Verfahren umfasst die Schritte
- Inkontaktbringen einer Probe, die die Nukleinsäuren in einer Flüssigkeit enthält, mit den magnetischen Partikeln mit Glasoberflächen unter Bedingungen, bei denen die Nukleinsäuren in nativer Form an die Glasberfläche binden können,
- Binden der Nukleinsäuren in nativer Form durch Adsorption an die Glasoberfläche der magnetischen Partikel,
- Abtrennung der Nukleinsäuren von der Flüssigkeit und
- Einsetzen der Nukleinsäuren als Substrat in einer enzymatischen Reaktion.

Unter biologischen Materialien werden Nukleinsäuren, z.B. DNA oder RNA, verstanden.

Proben im Sinne der Erfindung sind beispielsweise klinische Proben, wie Blut, Serum, Mundspülflüssigkeit, Urin, Zerebralflüssigkeit, Sputum, Stuhl, Punktate und Knochenmarkproben. Die Probe kann auch aus dem Bereich der Umweltanalytik, der Lebensmittelanalytik oder der molekularbiologischen Forschung, z. B. aus Bakterienkulturen, Phagenlysaten und Produkten von Amplifikationsverfahren, z. B. PCR, stammen.

Die beschriebenen magnetischen Partikel haben einen inneren Kern, auf den die äußere Glasoberfläche aufgebracht ist. Bei dem Kern kann es sich um einen Verbundstoff, jedoch auch um einfache Eisenkerne handeln. Der Kern kann auch aus einer kristallinen oder keramischen oder glasartigen Struktur bestehen, in die Eisenoxid eingelagert ist.

Mit dem geschilderten Verfahren können native oder modifizierte Nukleinsäuren isoliert werden. Unter nativen Nukleinsäuren werden solche verstanden, deren Struktur gegenüber den natürlich vorkommenden Nukleinsäuren nicht irreversibel verändert wurde. Dies schließt jedoch nicht die Modifizierung anderer Bestandteile der Probe aus. So kann zwar das die Zellen umgebende Medium modifiziert sein, nicht jedoch die Nukleinsäuren als solche. Die Nukleinsäuren sollen in der nativen Form, d.h. nicht denaturiert, geschnitten oder durch Ankoppelung reaktiver Gruppen modifiziert sein. Der Begriff native Nukleinsäuren umfasst daher biotinylierte Nukleinsäuren nicht. Beispiele für native Nukleinsäuren sind Phagen-DNA oder zelluläre Nukleinsäuren aus Blut.

Modifizierte Nukleinsäuren umfassen solche, die nicht in der Natur vorkommen, z.B. Nukleinsäuren, die durch Anheftung reaktiver, nachweisbarer oder zur Immobilisierung befähigenden Gruppen modifiziert sind, z.B. biotinylierte Nukleinsäuren.

In bestimmten Fällen kann die Probe ohne Vorbehandlung in das Isolierungsverfahren eingesetzt werden. In vielen Fällen sollte die Probe jedoch durch eine geeignete Methode aufgeschlossen und die in der Probe enthaltenen Nukleinsäuren freigesetzt werden. Verfahren zum Aufschluss von Proben sind dem Fachmann bekannt und können chemischer, enzymatischer oder physikalischer Natur sein. Auch eine Kombination dieser Verfahren ist möglich. Beispielhaft genannt sei Lyse durch Ultraschall, Hochdruck oder durch Scherung, durch Alkali, Detergenzien oder chaotrope Salzlösungen, oder durch Einwirkung von Proteinasen oder Lipasen. Speziell im Hinblick auf die Aufschlussverfahren zum Erhalt von Nukleinsäuren wird auf Sambrook et al.: Molecular Cloning, A Laboratory Manual, 2nd Addition, Cold Spring Harbour Laboratory Press, Cold Spring Harbour, NY und Ausubel et al.: Current Protocols in Molecular Biology 1987, J. Viley and Sons, NY, verwiesen.

Die Probe kann neben den zu isolierenden Nukleinsäuren weitere Bestandteile, z. B. Zelltrümmer, Proteine, Salze und weitere nicht zu isolierende Stoffe in einer Flüssigkeit enthalten. Diese Probe, die die Nukleinsäuren in nativer Form enthält, wird unter Bedingungen, bei denen die gewünschten Nukleinsäuren an die Partikeloberfläche binden, mit den Partikeln in Kontakt gebracht. Die Bedingungen hierfür sind prinzipiell bekannt. Sie richten sich nach der Art der Bindung, über die die Nukleinsäuren an die Oberfläche gebunden werden. Im Fall der modifizierten Nukleinsäuren ist eine Bindung über die Gruppen der Nukleinsäuren möglich, die die Modifizierung darstellen, z. B. Biotin über die Bindung an mit Streptavidin beschichtete Oberflächen. Erfindungsgemäß ist jedoch der Fall der direkten Bindung von Nukleinsäuren an Glas. Die Bindung nativer Nukleinsäuren an Glaspartikel kann analog zu Verfahren des Standes der Technik erfolgen. Bevorzugt erfolgt sie in Gegenwart chaotroper Salze, wobei die Konzentration dieser Salze zwischen 2 und 8 mol/l beträgt, bevorzugt 4 bis 6 mol/l. Bei chaotropen Salzen handelt es sich z. B. um Natriumjodit, Natriumperchlorat, Guanidininiumthiocyanat, Guanidiniumisothiocyanat oder Guanidiniumhydrochlorit, ist jedoch nicht auf diese Verbindungen beschränkt.

Zum Inkontaktbringen der Probe mit den Partikeln wird die Probe mit den Partikeln vermischt und für eine für die Bindung ausreichende Zeit inkubiert. Die Länge der Inkubation ist dem Fachmann in der Regel aus der Behandlung mit nicht-magnetischen Partikeln bekannt, eine Optimierung ist durch Durchführung einer Bestimmung der Menge an immobilisierten Nukleinsäuren an der Oberfläche zu verschiedenen Zeitpunkten möglich. Es können Inkubationszeiten zwischen 10 Sekunden und 30 Minuten zweckmäßig sein.

Je nach Größe und Art der magnetischen Partikel findet schon während der Inkubationszeit eine Separation der Partikel von der Flüssigkeit statt oder erhält sich die Suspension über längere Zeit. Wenn die Partikel eine sehr kleine Korngröße aufweisen und superparamagnetisch sind, erhält sich die Suspension über einen längeren Zeitraum. Handelt es sich um Partikel mit einer größeren Korngröße, so findet bereits während der Inkubation eine langsame Separation der Partikel von der Flüssigkeit statt. Insbesondere wenn es sich um ferromagnetische Partikel handelt, bilden sich solche Aggregate. Für den bevorzugten Fall, dass die ferromagnetischen Partikel nicht vormagnetisiert sind, ist eine besonders schonende Separation gewährleistet.

Die Immobilisierung findet nicht durch Ausfällung durch Erniedrigung der Löslichkeit der zu immobilisierten Nukleinsäuren statt. Stattdessen beruht die Immobilisierung auf Adsorption. Dies vermeidet weitgehend unspezifische Einschlüsse von Verunreinigungen.

Nach der Inkubation erfolgt die Abtrennung der Nukleinsäuren von der Flüssigkeit. Dies wird allgemein durch die Separation der an die magnetischen Partikel gebundenen Nukleinsäuren mit Hilfe eines Magnetfeldes erreicht. Beispielsweise können die Magnetpartikel an die Wand des Gefäßes, in welchem die Inkubation stattgefunden hatte, gezogen werden. Daraufhin kann die Flüssigkeit mit den Inhaltsstoffen der Probe, die nicht an die magnetischen Partikel gebunden wurden, entfernt werden. Diese Entfernung hängt von der Art des Gefäßes ab, in dem die Inkubation stattgefunden hat. Geeignete Verfahrensschritte sind Abpipettieren oder Absaugen der Flüssigkeit.

Danach können die magnetischen Partikel gewünschtenfalls ein- oder mehrmals mit einer Waschlösung gereinigt werden. Die Waschlösung wird so gewählt, dass eine Ablösung der Nukleinsäuren von der Partikeloberfläche möglichst nicht stattfindet, jedoch nicht zu isolierende Verunreinigungen möglichst gut weggewaschen werden. Dieser Waschschritt findet bevorzugt durch Inkubation der Waschlösung mit den Partikeln statt, wobei bevorzugt eine Resuspension der Partikel vorgenommen wird, z. B. durch Schütteln oder Anlegung eines nicht mit dem ersten Magnetfeld identischen Magnetfeldes. Die verunreinigte Waschlösung wird bevorzugt genauso entfernt wie die Probe in dem oben genannten Schritt zur Bindung der Nukleinsäuren.

Im Anschluss an den letzten Waschschritt kann ein kurzer Trocknungsschritt der magnetischen Partikel im Vakuum oder durch Ausdampfen (lassen) der Flüssigkeit vorgenommen werden, wobei auch eine Vorbehandlung mit Aceton möglich ist.

Die so gereinigten Nukleinsäuren können, falls gewünscht, von den magnetischen Partikeln entfernt werden. Da es sich um native Nukleinsäuren und bei den magnetischen Partikeln um glasüberzogene Partikel handelt, kann die Nukleinsäure mittels eines Elutionspuffers mit niedrigem Salzgehalt von den Partikeln entfernt werden. Solche Puffer sind aus DE 3724442 und Analytical Biochemistry 175, 196-201 (1988) bekannt. Als Elutionspuffer mit niedrigem Salzgehalt werden insbesondere Puffer mit einem Gehalt von weniger als 0,2 mol/l eingesetzt. In einer besonders bevorzugten Ausführungsform enthält der Elutionspuffer Tris. In einer anderen besonderen Ausführungsform handelt es sich bei dem Elutionspuffer um entmineralisiertes Wasser.

In einer weiteren Ausführungsform kann das beschriebene Reinigungs- und Isolierungsverfahren im Anschluss an eine immunomagnetische Separation von Zellen (z. B. virale Partikel oder prokaryontische bzw. eukaryontische Zellen) aus einer Körperflüssigkeit oder einem Gewebe erfolgen. Hierzu wird die Probe mit magnetischen Partikeln, an welche ein Antikörper gegen ein Antigen auf der Zelle immobilisiert ist, z. B. unter Schütteln inkubiert. Solche Partikel können die oben beschriebenen Partikel sein, aber auch käufliche (z. B. MACS Microbeads der Firma Miltenyi Biotec GmbH, Bergisch Gladbach, BRD). Nach Anlegen eines Magnetfeldes erfolgen ein oder mehrere Waschschritte mit einer salzhaltigen Waschlösung. Man erhält Partikel, an welche die gewünschten Zellen gebunden sind. Zur Isolierung der Nukleinsäuren werden die gebundenen Zellen in einem salzhaltigen Puffer resuspendiert. In einer bevorzugten Ausführungsform ist dieser salzhaltige Puffer eine chaotrope Salzlösung, so dass die in der Zelle vorhandenen Nukleinsäuren aus den Zellen freigesetzt werden.

Durch Kombination der oben beschriebenen Isolierung von Zellen mit der ebenfalls beschriebenen Isolierung von Nukleinsäuren in ihrer nativen Form an den magnetischen Partikeln, ergibt sich ein besonders vorteilhaftes Verfahren zur Isolierung von Nukleinsäuren aus zellhaltigen Proben zur anschließenden enzymatischen Reaktion. Vorteil dieser Ausführungsform ist die mögliche Einfachheit (Single-Tube-Methode), hohe Sensitivität (besonders wichtig in der medizinischen Mikrobiologie und Onkologie) und die leichte Automatisierbarkeit.

Die isolierten Nukleinsäuren können nun in beliebiger Weise als Substrat für verschiedene enzymatische Reaktionen verwendet werden. Als Beispiel seien die Sequenzierung, die radioaktive oder nicht-radioaktive Markierung, die Amplifikation einer oder mehrerer in ihr enthaltender Sequenzen, die Transkription, die Hybridisierung mit markierten Sondennukleinsäuren, die Translation oder die Ligation genannt. Ein Vorteil des erfindungsgemäßen Verfahrens ist, dass die Abtrennung der Nukleinsäuren von der Flüssigkeit sehr einfach ist. Im Stand derTechnik wurde zur Separierung von Glaspartikeln von Verunreinigungen entweder ein Zentrifugationsschritt angewandt, oder im Falle der Bindung der Nukleinsäuren an Glasfiberfilter die Flüssigkeit durch diese Flüssigkeit gesaugt. Hierbei handelt es sich um einen limitierenden Schritt, der die Verarbeitung von großen Probenzahlen behindert.

Mit den beschriebenen Partikeln ist eine effektivere Abtrennung der Nukleinsäuren von Verunreinigungen möglich. Insbesondere können Inhibitoren für bestimmte enzymatische Reaktionen in besonders gutem Umfang entfernt werden. Die Ausbeute an Nukleinsäuren ist vergleichsweise hoch. Eine Fraktionierung langer Nukleinsäuren wurde nicht beobachtet. Die beschriebenen Partikel sind bevorzugt schneller magnetisierbar.
In Figur 1 ist schematisch eine Isolierung von Nukleinsäuren aus einer zellhaltigen Probe gezeigt.
In Figur 2 ist die Auftrennung isolierter Nukleinsäuren in einem Agarosegel gezeigt.
In Figur 3 ist die Auftrennung von Reaktionsprodukten nach erfindungsgemäßer Isolierung und PCR-Amplifikation dargestellt.
In Figur 4 ist ein Gel der Ergebnisse aus Beispiel 4 gezeigt.

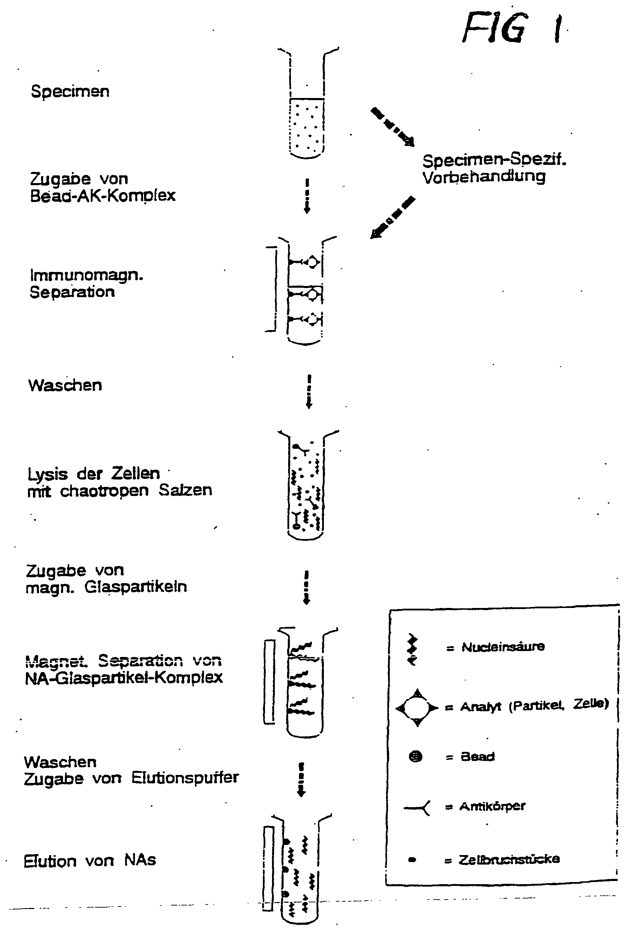

in Figur 1 ist eine Isolierung vor Nukleinsäuren aus einer zellhaltigen Probe schematisch dargestellt. Die Probe (Specimen), welche Zellen enthält, wird probenspezifisch so vorbehandelt, dass die Zellen, in denen die Nukleinsäuren nachgewiesen werden sollen, in geeigneter Form vorliegen. Hierzu gehört z. B. bei Proben, welchen Körperflüssigkeiten entnommen wurden, die Zugabe von Reagenzien, z. B. zur Verflüssigung von zähflüssigen Proben, z. B. Speichelproben. Der so vorbereiteten Probe wird in einem Gefäß ein an eine Festphase, bevorzugt an eine Perle (Bead) gebundener Antikörper zugegeben, welcher die Zelle erkennen und binden kann. Als geeignete Partner für den Antikörper haben sich beispielsweise Antigene auf der Zelloberfläche erwiesen. Die Spezifität des Antikörpers kann sich nach der Spezifität der zu lösenden Analyseaufgabe richten. Wenn es sich bei der Festphase um die Wand des Gefäßes handelt, werden die Zellen direkt an die Wand gebunden. Für den Fall, dass es sich bei den Festphasen um eine Perle handelt, werden diese durch geeignete Separationsmethoden von der Flüssigkeit separiert. Dies kann beispielsweise durch Filtration geschehen. Im Falle von magnetischen Perlen ist eine Abtrennung durch Anlegen eines Magnetfeldes an die Außenwand des Gefäßes möglich. Die separierten Zellen werden mit einer Flüssigkeit gewaschen, um Verunreinigungen, welche den Nachweis stören würden, mit dem die Zellen umgebenden Medium zu entfernen. Bevorzugt werden Bedingungen eingesetzt, bei denen die Zellen weder von der Festphase gelöst noch zerstört werden. Anschließend findet die Zerstörung der Zellen statt, die sogenannte Lyse. Eine Möglichkeit ist durch die Behandlung der Zellen mit chaotropen Salzen gegeben. Andere Möglichkeiten sind die Einwirkung von Proteinasen und Detergenzien.

Zu der Lysemischung werden in der bevorzugten Ausführungsform die Partikel zugegeben. Nach einer geeigneten Einwirkungszeit, die über die Beladung der Oberfläche mit Nukleinsäuren optimiert werden kann, werden die Partikel von der sie umgebenden Flüssigkeit, die weitere und nicht nachzuweisende Zellbestandteile enthält, getrennt. Dies geschieht wiederum bevorzugt durch Anlegen eines magnetischen Feldes mittels eines Magneten an der Gefäßwand.

Um eventuell noch anhaftende Verunreinigungen zu entfernen, wird bevorzugt mit einer Flüssigkeit gewaschen, die so ausgewählt wird, dass die zu bestimmenden Nukleinsäuren sich nicht von der Glasoberfläche ablösen.

Zur Entfernung der Nukleinsäuren von der Glasoberfläche wird ein sogenannter Elutionspuffer zugegeben, der Reagenzbedingungen aufweist, unter denen sich die Nukleinsäuren von der Glasoberfläche lösen. Dies sind insbesondere Niedrigsalzbedingungen. Je nach beabsichtigter Weiterbehandlung der Nukleinsäuren kann die Flüssigkeit nun von den Partikeln getrennt und weiterbearbeitet werden. Es ist bevorzugt, diese Abtrennung bei anliegendem Magnetfeld vorzunehmen, so dass die Partikel separiert vorliegen.

Die folgenden Beispiele erläutern die Erfindung näher.

### Beispiel 1

### Herstellung der magnetischen Partikel

Es wurden 6 verschiedene Sole verwendet. Die Herstellung der Sole wurde nach folgenden Schemata durchgeführt:

Sol 1 (SiO₂:B₂O₃ = 7:3):

Die Synthese wurde in einem 250 ml Rundkolben unter ständigem Rühren durchgeführt. 86,6 ml Tetraethylorthosilicat
+ 7 ml wasserfreies unvergälltes Ethanol
+ 14,1 ml 0,15 M HCl

Es entsteht ein zweiphasiges Gemisch, welches bei Raumtemperatur solange gerührt wird, bis es einphasig wird. Danach erfolgt tropfenweise die Zugabe von
+ 37,8 ml Trimethylborat.
Anschließend wird das Sol 2 Stunden bei 50 °C gehalten. Danach erfolgt die Zugabe von
+ 14,1 ml 0,15 M HCl

Sol 2 (SiO₂:B₂O₃=4:1):

Die Synthese wurde in einem 250 ml Rundkolben unter ständigem Rühren durchgeführt. 100,5 ml Tetraethylorthosilicat
+ 7 ml wasserfreies unvergälltes Ethanol
+ 16, 3 ml 0,15 M HCl

Es entsteht ein zweiphasiges Gemisch, welches bei Raumtemperatur solange gerührt wird, bis es einphasig wird. Danach erfolgt tropfenweise die Zugabe von + 25,6 ml Trimethylborat.
Anschließend wird das Sol 2 Stunden bei 50 °C gehalten. Danach erfolgt die Zugabe von + 16,3 ml 0,15 M HCl

Sol 3 (SiO₂:B₂O₃ = 85:15):

Die Synthese wurde in einem 250 ml Rundkolben unter ständigem Rühren durchgeführt. 107,8 ml Tetraethylorthosilicat
+ 7 ml wasserfreies unvergälltes Ethanol
+ 17,5 ml 0,15 M HCl

Es entsteht ein zweiphasiges Gemisch, welches bei Raumtemperatur solange gerührt wird, bis es einphasig wird. Danach erfolgt tropfenweise die Zugabe von + 19,4 ml Trimethylborat.
Anschließend wird das Sol 2 Stunden bei 50 °C gehalten. Danach erfolgt die Zugabe von + 17,5 ml 0,15 M HCl

Sol 4 (SiO₂:B₂O₃ = 4:1; 2 Mol% P₂O₅):

Die Synthese wurde in einem 250 ml Rundkolben unter ständigem Rühren durchgeführt. 100,5 ml Tetraethylorthosilicat
+ 7 ml wasserfreies unvergälltes Ethanol
+ 16,3 ml 0,15 M HCl

Es entsteht ein zweiphasiges Gemisch, welches bei Raumtemperatur solange gerührt wird, bis es einphasig wird. Danach erfolgt tropfenweise die Zugabe von
+ 25, 6 ml Trimethylborat
Anschließend wird das Sol 2 Stunden bei 50 °C gehalten. Danach erfolgt die Zugabe von + 16,3 ml 0,15 M HCl
+ 1,63 g P₂O₅

Sol 5 (SiO₂:B₂O₃ = 4:1 Mol% Al₂O₃):

Die Synthese wurde in einem 250 ml Rundkolben unter ständigem Rührem durchgeführt. 100,5 ml Tetraethylorthosilicat
+ 7 ml wasserfreies unvergälltes Ethanol
+ 16,3 ml 0, 15 M HCl

Es entsteht ein zweiphasiges Gemisch, welches bei Raumtemperatur solange gerührt wird, bis es einphasig wird. Danach erfolgt tropfenweise die Zugabe von
+ 25,6 ml Trimethylborat.
Anschließend wird das Sol 2 Stunden bei 50 °C gehalten. Danach erfolgt die Zugabe von + 16,3 ml 0,15 M HCl
+ 3,06 g AlCl₃
Sol 6 (SiO₂:B₂o₃ = 4:1 Mol 1% ZrO₂):

Die Synthese wurde in einem 250 ml Rundkolben unter ständigem Rühren durchgeführt. 100,5 ml Tetraethylorthosilicat
+ 7 ml wasserfreies unvergälltes Ethanol
+ 16,3 ml 0,15 M HCl

Es entsteht ein zweiphasiges Gemisch, welches bei Raumtemperatur solange gerührt wird, bis es einphasig wird. Danach erfolgt tropfenweise die Zugabe von
+ 25,6 ml Trimethylborat
+ 5,15 ml Zirkon(IV)-propylat, 70 Gew.% Lsg in 1-Propanol
Anschließend wird das Sol 2 Stunden bei 50 °C gehalten. Danach erfolgt die Zugabe von + 16,3 ml 0,15 M HCl

Nach weiteren 2 Stunden bei 50 °C wurde in jeweils 150 ml der Sole 22,5 g Iriodin 600 (Black Mica) eingerührt und anschließend mit einem Sprühtrockner (Büchi 190, Mini Spray Dryer) beschichtet. Die Düsentemperatur des Sprühtrockners betrug 134 °C.

Das durch den Sprühtrockenprozess erhaltene Pulver wurde anschließend einer Temperaturbehandlung unter Stickstoffatmosphäre (90 l/h) unterzogen. Die Aufheizgeschwindigkeit betrug hierbei 1 k/min und die Haltezeit betrug 2 Stunden bei der Verdichtungstemperatur. Diese Temperatur lag bei der Beschichtung mit Sol 1 bei 750 °C, bei der Beschichtung mit Sol 2 bei 860 °C und bei den übrigen Beschichtungen bei 800 °C. Nach dem Sinterprozess wurde der Ofen abgeschaltet und das Pulver auf Raumtemperatur abgekühlt. Eventuell entstandene Agglomerate wurden mit einem 50 µm Sieb ausgesiebt.

### Beispiel 2

### Herstellung von GMP1, GMP2, GMP3 und GMP4

GMP1, GMP2, GMP3 und GMP4 sind Pigmente aus unterschiedlichen Herstellungs-Chargen, die aus Sol 1 aus Beispiel 1 in einem Prozess nach Beispiel 1 unter folgenden Bedingungen erhalten wurden:

| **Parameter** | **GMP1** | **GMP2** | **GMP3** | **GMP4** |
|---|---|---|---|---|
| Alterung des Sols (h) (30 °C) | 36 | 36 | 36 | 36 |
| Pigmentanteil des Sols (g/100 ml) | 5 | 15 | 8 | 20 |
| Luftstrom der Düse (%) | 100 | 100 | 100 | 100 |
| Luftdruck (bar) | 6 | 6 | 6 | 3 |
| Düsentemperatur (°C) | 135 | 120 | 130 | 143 |
| Verdichtungstemperatur (°C) | 534 | 534 | 534 | 615 |
| 02-Nachbehandlung (1 Stunde) | (300 °C) | (300 °C) | 300 °C) | (400 °C) |
| Ausbeute an Pigment | niedrig | hoch | mittel | hoch |
| DNA-Ausbeute | niedrig | hoch | hoch | hoch |

### Beispiel 3

### PCR Probenvorbereitung aus humanen Vollblut mit magnetischen Glaspartikeln

### Isolierung der Nukleinsäure

Von drei Glasmagnetpartikel-Chargen (GMP2 - 4) wurden je ca. 10 mg in Eppendorf Reaktionsgefäßen vorgelegt. Die genauen Einwaagen sind in Tabelle 1 angegeben, es wurden Dreifach-Bestimmungen durchgeführt.

Zu je 200 µl aufgetautem Vollblut wurden 40 µl Proteinase K (20 mg/ml, hergestellt aus Lyophilisat) pipetiert und sofort gemischt. Anschließend wurden 200 µl Bindepuffer (6 M Guanidin-HCl, 10 mM Tris-HCl, 10 mM Harnstoff, 30 % Triton X-100, pH 4,4) zugegeben, gemischt und für 10 Minuten bei 70 °C inkubiert. Nach Zugabe von 200 µl i-Propanol wurde für 10 Sekunden auf dem Vortex-Mischer gemischt, die Probe für 20 Minuten bei Raumtemperatur inkubiert und abermals für 10 Sekunden wie vor gemischt. Die Magnetseparation erfolgte für wenigstens 30 Sekunden im Magnetpartikelseparatorvon Boehringer Mannheim (Id.-Nr. 1 641 794). Der Überstand wurde abgenommen und wie weiter unten beschrieben analysiert.

Die Magnetpartikel wurden mit jeweils 500 µl Wasch-Puffer (20 mM NaCl, 10mM Tris-HCl, pH 7,5 (25 °C), 80 % Ethanol) durch 10 Sekunden mischen, 1 Minute Inkubation bei Raumtemperatur und 10 Sekunden mischen gewaschen und mit dem Magnetpartikelseparator an die Gefäßwand gezogen. Der Überstand wurde abgenommen und verworfen. Die Waschprozedur wurde wiederholt bis der Waschüberstand farblos war (insgesamt 4 x Waschen). Nun wurden die Nukleinsäuren 3 x mit jeweils 200 µl auf 70 °C vorgewärmten Elutionspuffer (10 mM Tris-HCl, pH 8,5) durch 10 Sekunden mischen, 10 Minuten Inkubation bei Raumtemperatur und 10 Minuten mischen eluiert.

### Aufarbeitung des Überstandes

Der Überstand nach der 1. Bindung an die magnetischen Glaspartikel wurde folgendermaßen auf Gehalt an Nukleinsäuren überprüft: Der Überstand wurde in ein Filter-Tube (BoehringerMannheim, Id.-Nr. 1744003, z. B. enthalten in High Pure PCR Product Purification Kit) gegeben und für 1 Minute bei 8000 rpm in einer Eppendorf Tischzentrifuge zentrifugiert. Der Durchlauf wird verworfen und das Filter-Tube 2 x mit 500 µl Wasch-Puffer gewaschen (Zentrifugation wie vor). Das Filter-Tube wird kurz trocken zentrifugiert und dann mit 2 x 200 µl auf 70 °C vorgewärmten 1 x Elutionspuffer durch erneute Zentrifugation eluiert.

### Analyse der Eluate und des Probenüberstandes

50 µl der Eluate bzw. der über Filter-Tube ausgearbeiteten Überstände wurden mit 10 µl Proben-Puffer versetzt und davon 45 µl in einem 0,8 %igen Agarosegel elektrophoretisch bei 120 V für 90 Minuten aufgetrennt.

Verschiedene Verdünnungen der Eluate bzw. der aufgearbeiteten Überstände wurden bei 260 und 280 nm in einem Uvikon 710 (Kontron) spektroskopisch vermessen.

Zwei 5 µl Aliquots der Eluatewurden als Doppelbestimmungdurch Expand TM LongTemplate PCR (Boehringer Mannheim, Id.-Nr. 1681834) mit spezifischen Primem für das menschliche tPA-Gen (erwartete Produktlänge 15 kb) überprüft.

| Mix I | pro Ansatz | Mix II | pro Ansatz |
|---|---|---|---|
| dNTP, je 100 mM | 1 µl | Expand™ Puffer, 10 x | 5 µl |
| Primer 1, 200 ng/µl | 1 µl | Expand™ Polymerase | 0,75 µl |
| Primer 2, 225 ng/µl | 1 µl | H₂O_{bidest} | 19,25 µl |
| H₂O_{bidest.} | 17 µl | | |
| | 20 µl | | 25 µl |

Mix I wird in ein dünnwandiges PCR-Tube vorgelegt mit 5 µl Eluat versetzt und Mix II zugegeben. Der Ansatz wird kurz gemischt und mit 30 µl Mineralöl überschichtet. Die Ansätze werden in einem Perkin-Elmer Thermocycler 9600 mit folgenden Programm amplifiziert:

| | | |
|---|---|---|
| 2 Minuten | 92 °C | |
| | | |
| 10 Sekunden | 92 °C | |
| 30 Sekunden | 65 °C | 10 Zyklen |
| 12 Minuten | 68°C | |
| | | |
| 10 Sekunden | 92 °C | |
| 30 Sekunden | 65 °C | 20 Zyklen |
| 12 Minuten | 68 °C | |
| +20 Sekunden pro Zyklus | | |
| | | |
| 7 Minuten | 68 °C | |
| anschließend | 7 °C | |

Die 50 µl PCR Ansätze wurden mit 10 µl Proben-Puffer versetzt und davon 45 µl in einem 0,8 %igen Agarosegel elektrophoretisch bei 120 V für 90 Minuten aufgetrennt.

### Ergebnisse

**Tabelle 1: Ausbeute an Nukleinsäuren mit magnetischen Glaspartikeln aus 200 µl Blut**

| | | Überstand 1:8 | | | | | 1. Eluat 1:8 | | | |
|---|---|---|---|---|---|---|---|---|---|---|
| | | 260 nm | 280 nm | Ausbeute | 260/280 | 260 nm | 280 nm | Ausbeute | 260/280 | |
| GMP/2 | 1 | 0,021 | 0,013 | 1,7 µg | 1,6 | 0,171 | 0,164 | 13,7 µg | 1,0 | |
| 12 mg | | | | | | | | | | |
| 10 mg | 2 | 0,045 | 0,035 | 3,7 µg | 1,3 | 0,137 | 0,138 | 11,0 µg | 1,0 | |
| 9 mg | 3 | 0,036 | 0,027 | 2,9 µg | 1,3 | 0,153 | 0,164 | 12,2 µg | 0,9 | |
| GMP/3 | 1 | 0,050 | 0,042 | 4,0 µg | 1,2 | 0,245 | 0,246 | 19,6 µg | 0,9 | |
| 10mg | | | | | | | | | | |
| 10 mg | 2 | 0,033 | 0,022 | 2,6 µg | 1,5 | 0,397 | 0,398 | 31,8 µg | 1,0 | |
| 10 mg | 3 | 0,042 | 0,030 | 3,4 µg | 1,4 | 0,278 | 0,282 | 22,2 µg | 0,9 | |
| GMP/4 | 1 | 0,065 | 0,056 | 0,7 µg | 1,2 | 0,135 | 0,142 | 11,0 µg | 1,0 | |
| 10 mg | | | | | | | | | | |
| 11 mg 2 | 2 | 0,071 | 0,142 | 2,4 µg | 0,5 | 0,140 | 0,142 | 11,2 µg | 1,0 | |
| 10mg | 3 | 0,066 | 0,051 | 1,7 µg | 1,3 | 0,130 | 0,130 | 10,4 µg | 1,0 | |

| | 2. Eluat 1:8 | | | | 3. Eluat 1:4 | | | | | |
|---|---|---|---|---|---|---|---|---|---|---|
| | | 260 nm | 280 nm | Ausbeute | 260/280 | 260 nm | 280 nm | Ausbeute | 260/280 | ∑Eluate |
| GMP/2 | 1 | 0,099 | 0,101 | 7,9 µg | 1,0 | 0,057 | 0,062 | 2,3 µg | 0,9 | 23,9 µg |
| | 2 | 0,078 | 0,076 | 6,2 µg | 1,0 | 0,041 | 0,049 | 1,6 µg | 0,8 | 18,8 µg |
| | 3 | 0,103 | 0,112 | 8,2 µg | 0,9 | fehlt | | | | |
| GMP/3 | 1 | 0,147 | 0,147 | 11,8 µg | 1,0 | 0,084 | 0,098 | 3,4 µg | 0,9 | 34,8 µg |
| | 2 | 0,256 | 0,252 | 20,5 µg | 1,0 | 0,042 | 0,043 | 1,7 µg | 1,0 | 54,0 µg |
| | 3 | 0,147 | 0,143 | 11,8 µg | 1,0 | 0,073 | 0,093 | 2,9 µg | 0,8 | 36,9 µg |
| GMP/4 | 1 | 0,106 | 0,108 | 8,5 µg | 1,0 | 0,083 | 0,098 | 3,3 µg | 0,8 | 22,8 µg |
| | 2 | 0,111 | 0,114 | 8,9 µg | 1,0 | 0,054 | 0,063 | 2,2 µg | 0,9 | 22,3 µg |
| | 3 | 0,135 | 0,141 | 10,8 µg | 1,0 | 0,077 | 0,095 | 3,1 µg | 0,8 | 24,3 µg |

Die 1. Eluate waren noch leicht gelb gefärbt und teilweise mit feinen Magnetpartikeln kontaminiert.

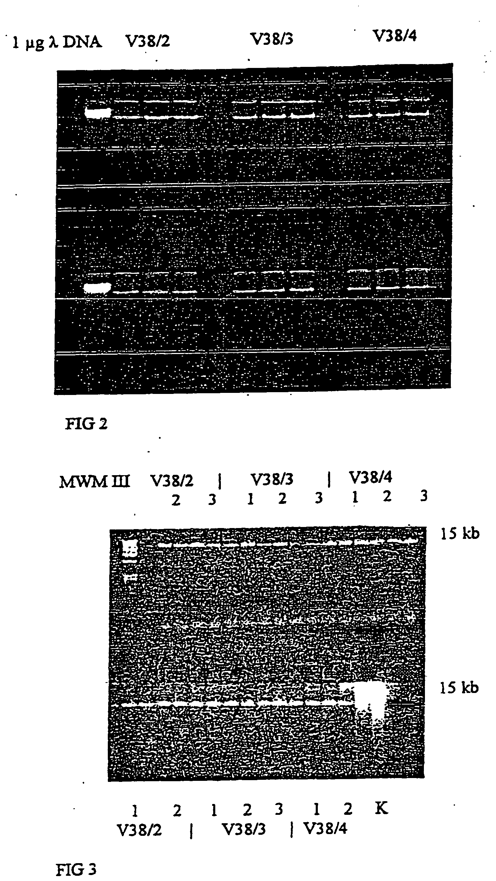

Die Analyse der Eluate im Agarosegel (FIG. 2) zeigt eine gute Reproduzierbarkeit der Ausbeute. Die Magnetpartikel GMP/2 - 4 zeigen keine signifikanten Unterschiede. In den Eluaten 1 (oben) und 2 (unten) ist etwa die gleiche Nukleinsäurekonzentration vorzufinden (vom Gel geschätzt). Eluat 3 zeigt nur noch eine geringe Nukleinsäurekonzentration. In den Überständen ist ebenfalls nur eine geringe Nukleinsäurekonzentration zu beobachten.

Die Expand™ PCR liefert mit allen Proben, bis auf wenige Ausreißer (Tabelle 2), durchweg gute und spezifische Amplifikationsprodukte. Mit den magnetischen Glasbeads ließen sich aus humanen Blutproben Nukleinsäuren isolieren, die in einer anschließenden PCR spezifische Amplifikate lieferten.

**Tabelle 2. Ergebnisse Expand™ PCR**

| | 15 kb Expand™ PCR | | humanes tPA Gen | |
|---|---|---|---|---|
| | 1. Eluat | | 2. Eluat | |
| GMP/2 1 | fehlt | | + | + |
| 2 | + | + | + | + |
| 3 | + | + | fehlt | |
| GMP/3 1 | + | + | + | + |
| 2 | (+) | + | + | + |
| 3 | - | (+) | + | + |
| GMP/4 1 | + | + | + | + |
| 2 | + | + | + | (+)* |
| 3 | + | + | fehlt | |
| K, BM Kontroll DNA | | | | |
| * 3. Eluat | | | | |

In FIG. 3 ist ein Gel mit den Reaktionsprodukten nach PCR-Amplifikation gezeigt. MWM III ist ein Molekulargewichtsmarker (Eluat 1, oben; Eluat 2, unten).

### Beispiel 4

### Bindung von DNA-Längenstandard an magnetische Glaspartikel

### 1. Vorbereitung der magnetischen Glaspartikel

Von der Glasmagnetcharge GMP4 wurden 12 mg in Eppendorf-Reaktionsgefäße vorgelegt.

### 2. Lyse und Bindung

In einem 1,5 ml Eppendorf-Gefäß mit 12 mg magnetischen Glaspartikeln werden 900 µl Lysis-Puffer (4.6 M GuSCN, 45 mM Tris, 20 mM EDTA, pH 7,3) und 100 µl DNA-Probe, in der modellhaft DNA-Längenstandard III von Boehringer Mannheim (Katalog-Nr. 528552) eingesetzt wurde, 2 bis 10 sec. gemischt, bis eine homogene Suspension entsteht. Die Lösung wird 20 min bei Raumtemperatur inkubiert, wobei alle 5 min gemischt wird.

Die Magnetseparation erfogt für mindestens 15 sec in einem Magnetpartikelseparator. Der Überstand wird abpipettiert.

### 3. Waschen und Trocknen

Die magnetischen Glaspartikel werden zweimal mit Waschpuffer (5.2 M GuSCN, 50 mM Tris, pH 6.5) zweimal mit 70 % vorgekühltem Ethanol und einmal mit Aceton gewaschen, indem das Magnetfeld entfernt, 800 µl Lösung zupipettiert, 2 sec gemischt, 1 min bei RT inkubiert, das Magnetfeld angelegt und der Überstand schließlich abpipettiert wird.

Nach Entfernung des Acetons werden die Partikel 10 min bei 56 °C im Heizblock bei offenem Deckel getrocknet.

### 4. Elution der DNA

Die DNA wird mit 4x 50 µl Elutionspuffer (10mM Tris-HCl, 1 mM EDTA, pH 8.0) eluiert, indem 10 min unter mehrmaligem Schütteln bei 56 °C inkubiert wird und der DNA-haltige Überstand schließlich in ein neues Eppendorf-Gefäß transferiert wird.

### 5. Analyse der Eluate

Ein Fünftel des Eluatvolumens wurde mit Probenpuffer versetzt und die DNA auf einem 1 %igen Agarosegel bei 90 V aufgetrennt. Zur Bestimmung der Recovery wurde eine Verdünnungsreihe von DNA-Längenstandard III auf das gleiche Gel aufgetragen, die die in den Proben zu erwartenden DNA-Mengen enthält.

Die quantitative Auswertung erfolgte durch Scannen eines Polaroidphotos des Agarosegels. Hierbei wurde die Verdünnungsreihe des Standards als Kalibrator verwendet.

Die Ausbeute an DNA mit magnetischen Glaspartikeln ist in Tabelle 1 dargestellt

**Tabelle 1. Ausbeute an DNA-Längenstandard III mit magnetischen Glaspartikeln**

| Standar Nr. | DNA-Menge im Standard [ng] | Helligkeitsistens Standard (Meßwert) [rel. Einheiten] | Probe Nr | Pigment/Bead-Typ | Helligkeitsintens, Probe (Meßwert) [rel. Einheiten] | errechnete DNA-Menge auf Gel [ng] | errechnete DNA-Menge in Probe [ng] | Recovery [%] |
|---|---|---|---|---|---|---|---|---|
| 1 | 200 | 65 | 1 | GMP4 | 45 | 139 | 695 | 69,5 |
| 2 | 175 | 56 | 2 | GMP4 | 39 | 120 | 600 | 60,0 |
| 3 | 150 | 51 | | | | | | |
| 4 | 125 | 44 | | | | | | |
| 5 | 100 | 37 | | | | | | |
| 6 | 75 | 25 | | | | | | |
| 7 | 50 | 17 | | | | | | |
| 8 | 25 | 9 | | | | | | |
| 9 | 10 | 4 | | | | | | |

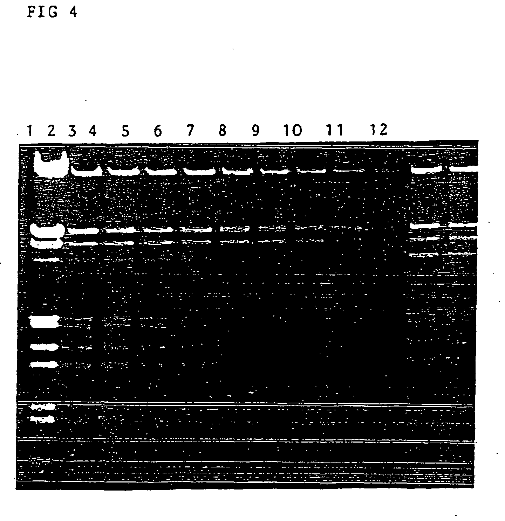

Das Agarosegel, das als Grundlage für die quantitative Auswertung diente, ist in FIG. 4 dargestellt. Es handelt sich um ein 1 %iges Ethidiumbromidgefärbtes Agarosegel. Spur 1 bis 10 entspricht einer Verdünnungsreihe von DNA-Längenstandard III. 1: 1 µg DNA, 2: 200 ng DNA, 3: 175 ng DNA, 4: 150 ng DNA, 5: 125 ng DNA, 6: 100 ng DNA, 7: 75 ng DNA, 8: 50 ng NDA, 9: 25 ng DNA, 10: 10 ng DNA.

Spur 11 und 12 entspricht der von den magnetischen Glaspartikeln eluierten DNA bei Einsatz von 200 ng DNA-Längenstandard.

### SEQUENZPROTOKOLL

<110> Roche Diagnostics GmbH
<120> Magnetisches Pigment
<130> 28921 PEP-WO_WWHC
<140>
   <141> >
<150> DE1952098.4
   <151> 1995-06-08
<150> DE19537985.3
   <151> 1995-10-12
<150> EP96921935.1
   <151> 1996-06-06
<160> 2
<170> Patent In Ver. 2.1
<210> 1
   <211> 34
   <212> DNA
   <213> Künstliche Sequenz
<220>
   <223> Beschreibung der künstlichen Sequenz: Oligodeoxyribonukleotid
<400> 1
   actgtgcttc ttgacccatg gcagaagcgc cttc 34
<210> 2
   <211> 34
   <212> DNA
   <213> Künstliche Sequenz
<220>
   <223> Beschreibung der künstlichen Sequenz: Oligodeoxyribonukleotid
<400> 2
   ccttcactgt ctgcctaact ccttcgtgtg ttcc PNTo<0:3>, PNTe<0:3>34

## Patentansprüche

1. Verfahren zur Isolierung von und enzymatischen Reaktion an Nukleinsäuren, umfassend
- Inkontaktbringen einer Probe, welche die Nukleinsäuren in einer Flüssigkeit enthält, mit magnetischen Partikeln mit Glasoberflächen unter Bedingungen, bei denen die Nukleinsäuren in nativer Form an die Glasoberfläche binden können,
- Binden der Nukleinsäuren in nativer Form durch Adsorption an die Glasoberfläche der magnetischen Partikel,
- Abtrennen der gebundenen Nukleinsäuren von der Flüssigkeit und
- Einsetzen der Nukleinsäuren als Substrat in einer enzymatischen Reaktion.

2. Verfahren nach Anspruch 1, enthaltend die Schritte:
(a) Adsorption von Nukleinsäuren in einer Probe, welche die Nukleinsäuren in einer Flüssigkeit enthält, an magnetische Partikel mit einer Glasoberfläche unter Bedingungen, unter welchen eine Bindung der Nukleinsäuren in nativer Form direkt an die Oberfläche stattfinden kann,
(b) Abtrennung der gebundenen Nukleinsäuren von der Flüssigkeit und anschließende Reinigung mit einer Waschlösung,
(c) gegebenenfalls Trocknung,
(d) Elution mit einem Elutionspuffer mit niedrigem Salzgehalt und
(e) Einsetzen der eluierten Nukleinsäuren als Substrat in einer enzymatischen Reaktion.

3. Verfahren nach Anspruch 1 oder 2, **dadurch gekennzeichnet, dass** es sich bei den Nukleinsäuren um DNA oder RNA handelt.

4. Verfahren nach einem der Ansprüche 1 bis 3, **dadurch gekennzeichnet, dass** bei der Isolierung Inhibitoren für enzymatische Reaktionen entfernt werden.

5. Verfahren nach einem der Ansprüche 1 bis 4, **dadurch gekennzeichnet, dass** bei der Isolierung eine Fraktionierung langer Nukleinsäuren nicht erfolgt.

6. Verfahren nach einem der Ansprüche 1 bis 5, **dadurch gekennzeichnet, dass** die magnetischen Partikel eine durchschnittliche Korngröße von weniger als 100 µm aufweisen.

7. Verfahren nach einem der Ansprüche 1 bis 6, **dadurch gekennzeichnet, dass** die magnetischen Partikel einen inneren Kern z.B. aus einem Verbundstoff oder einem Eisenkern enthalten, auf den die äußere Glasoberfläche aufgebracht ist.

8. Verfahren nach Anspruch 7, **dadurch gekennzeichnet, dass** der Kern aus einer kristallinen oder keramischen oder glasartigen Struktur besteht, in die Eisenoxid eingelagert ist.

9. Verfahren nach Anspruch 8, **dadurch gekennzeichnet, dass** das Eisenoxid Magnetit (Fe₃O₄) oder Fe₂O₃ ist.

10. Verfahren nach einem der Ansprüche 1 bis 9, **dadurch gekennzeichnet, dass** die magnetischen Partikel ferromagnetisch sind.

11. Verfahren nach einem der Ansprüche 1 bis 10, **dadurch gekennzeichnet, dass** die Isolierung der Nukleinsäuren in Gegenwart chaotroper Salze stattfindet.

12. Verfahren nach Anspruch 11, **dadurch gekennzeichnet, dass** die Konzentration chaotroper Salze 2-8 mol/l, vorzugsweise 4-6 mol/l beträgt.

13. Verfahren nach Anspruch 11 oder 12, **dadurch gekennzeichnet, dass** die chaotropen Salze ausgewählt werden aus Natriumjodid, Natriumperchlorat, Guanidiniumthiocyanat, Guanidiniumisothiocyanat und Guanidiniumhydrochlorid.

14. Verfahren nach einem der Ansprüche 1 bis 13, **dadurch gekennzeichnet, dass** die Probe mit den magnetischen Partikeln vermischt und für eine für die Bindung ausreichende Zeit inkubiert wird, vorzugsweise zwischen 10 Sekunden und 30 Minuten.

15. Verfahren nach Anspruch 14, **dadurch gekennzeichnet, dass** eine Abtrennung der Nukleinsäuren von der Flüssigkeit nach der Inkubation mit Hilfe eines Magnetfeldes erfolgt.

16. Verfahren nach einem der Ansprüche 1 bis 15, **dadurch gekennzeichnet, dass** die magnetischen Partikel ein- oder mehrmals mit einer Waschlösung gereinigt werden.

17. Verfahren nach Anspruch 16, **dadurch gekennzeichnet, dass** nach dem letzten Waschschritt ein Trocknungsschritt erfolgt, gegebenenfalls mit einer Vorbehandlung mit Aceton.

18. Verfahren nach Anspruch 16 oder 17, **dadurch gekennzeichnet, dass** die gereinigten Nukleinsäuren von den magnetischen Partikeln mit einem Elutionspuffer mit einem Salzgehalt von weniger als 0,2 mol/l eluiert werden.

19. Verfahren nach Anspruch 18, **dadurch gekennzeichnet, dass** der Elutionspuffer Tris enthält oder entmineralisiertes Wasser ist.

20. Verfahren nach einem der Ansprüche 1 bis 19, **dadurch gekennzeichnet, dass** als enzymatische Reaktion eine Sequenzierung, eine radioaktive oder nicht-radioaktive Markierung, eine Amplifikation einer oder mehrerer Sequenzen, eine Transkription, eine Hybridisierung mit markierten Sondennukleinsäuren, eine Translation oder eine Ligation durchgeführt wird.

## Claims

1. A method for isolation of and enzymatic reaction on nucleic acids comprising
- contacting a sample, which contains the nucleic acids in a liquid, with magnetic particles with a glass surface under conditions in which the nucleic acids can bind in native form onto the glass surface,
- binding the nucleic acids in native form by adsorption onto the glass surface of the magnetic particles,
- separating the bound nucleic acids from the liquid, and
- using the nucleic acids as substrate in an enzymatic reaction.

2. The method according to claim 1, containing the steps:
(a) adsorbing nucleic acids in a sample, which contains the nucleic acids in a liquid, onto magnetic particles with a glass surface under conditions in which the nucleic acids can bind in native form directly onto the surface of the particles,
(b) separating the bound nucleic acids from the liquid and then purifying with a wash solution,
(c) optionally drying,
(d) eluting with an elution buffer with low salt content, and
(e) using the eluted nucleic acids as substrate in an enzymatic reaction.

3. The method according to claim 1 or 2, **characterised in that** the nucleic acids are DNA or RNA.

4. The method according to any one of claims 1 to 3, **characterised in that** inhibitors of enzymatic reactions are removed during isolation.

5. The method according to any one of claims 1 to 4, **characterised in that** long nucleic acids are not fractionated during isolation.

6. The method according to any one of claims 1 to 5, **characterised in that** the magnetic particles have an average grain size of less than 100 µm.

7. The method according to any one of claims 1 to 6, **characterised in that** the magnetic particles contain an inner core, for example of a composite material or an iron core, onto which the outer glass surface is applied.

8. The method according to claim 7, **characterised in that** the core consists of a crystalline or ceramic or vitreous structure in which iron oxide is embedded.

9. The method according to claim 8, **characterised in that** the iron oxide is magnetite (Fe₃O₄ or Fe₂O₃.

10. The method according to any one of claims 1 to 9, **characterised in that** the magnetic particles are ferromagnetic.

11. The method according to any one of claims 1 to 10, **characterised in that** the isolation of the nucleic acids proceeds in the presence of chaotropic salts.

12. The method according to claim 11, **characterised in that** the concentration of chaotropic salts is 2-8 mol/l, preferably 4-6 mol/l.

13. The method according to claim 11 or 12, **characterised in that** the chaotropic salts are selected from sodium iodide, sodium perchlorate, guanidinium thiocyanate, guanidinium isothiocyanate and guanidinium hydrochloride.

14. The method according to any one of claims 1 to 13, **characterised in that** the sample is mixed with the magnetic particles and incubated for a period of time sufficient for binding to occur, preferably between 10 seconds and 30 minutes.

15. The method according to claim 14, **characterised in that** the nucleic acids are separated from the liquid after incubation with the aid of a magnetic field.

16. The method according to any one of claims 1 to 15, **characterised in that** the magnetic particles are purified once or more with a wash solution.

17. The method according to claim 16, **characterised in that** the last wash step is followed by a drying step, optionally with pre-treatment with acetone.

18. The method according to claim 16 or 17, **characterised in that** the purified nucleic acids are eluted from the magnetic particles with an elution buffer with a salt content of less than 0.2 mol/l.

19. The method according to claim 18, **characterised in that** the elution buffer contains Tris or is demineralised water.

20. The method according to any one of claims 1 to 19, **characterised in that** the enzymatic reaction carried out is sequencing, a radioactive or non-radioactive labelling, an amplification of one or more sequences, a transcription, a hybridisation with labelled probe nucleic acids, a translation or a ligation.

## Revendications

1. Procédé pour l'isolement d'acides nucléiques et la réaction enzymatique sur les acides nucléiques, comprenant
- la mise en contact d'un échantillon, qui contient les acides nucléiques dans un liquide, avec des particules magnétiques présentant une surface en verre dans des conditions dans lesquelles les acides nucléiques peuvent se lier sous forme native à la surface en verre,
- la liaison des acides nucléiques sous forme native par adsorption à la surface en verre des particules magnétiques,
- la séparation des acides nucléiques liés du liquide et
- l'utilisation des acides nucléiques comme substrat dans une réaction enzymatique.

2. Procédé selon la revendication 1, contenant les étapes :
(a) adsorption d'acides nucléiques dans un échantillon, qui contient les acides nucléiques dans un liquide, sur des particules magnétiques présentant une surface en verre dans des conditions dans lesquelles une liaison des acides nucléiques sous forme native peut se produire directement sur la surface,
(b) séparation des acides nucléiques liés du liquide et purification consécutive avec une solution de lavage,
(c) le cas échéant séchage,
(d) élution avec un tampon d'élution présentant une faible teneur en sel et
(e) utilisation des acides nucléiques élués comme substrat dans une réaction enzymatique.

3. Procédé selon la revendication 1 ou 2, **caractérisé en ce qu'**il s'agit, pour les acides nucléiques, d'ADN ou d'ARN.

4. Procédé selon l'une quelconque des revendications 1 à 3, **caractérisé en ce que** lors de l'isolement, des inhibiteurs de réactions enzymatiques sont éliminés.

5. Procédé selon l'une quelconque des revendications 1 à 4, **caractérisé en ce que** lors de l'isolement, un fractionnement d'acides nucléiques longs ne se produit pas.

6. Procédé selon l'une quelconque des revendications 1 à 5, **caractérisé en ce que** les particules magnétiques présentent une taille de particule moyenne inférieure à 100 µm.

7. Procédé selon l'une quelconque des revendications 1 à 6, **caractérisé en ce que** les particules magnétiques contiennent un noyau interne, par exemple en un composite ou un noyau en fer, sur lequel est appliquée la surface externe en verre.

8. Procédé selon la revendication 7, **caractérisé en ce que** le noyau est constitué par une structure cristalline ou céramique ou une structure vitreuse dans laquelle est incorporé de l'oxyde de fer.

9. Procédé selon la revendication 8, **caractérisé en ce que** l'oxyde de fer est de la magnétite (Fe₃O₄) ou du Fe₂O₃.

10. Procédé selon l'une quelconque des revendications 1 à 9, **caractérisé en ce que** les particules magnétiques sont ferromagnétiques.

11. Procédé selon l'une quelconque des revendications 1 à 10, **caractérisé en ce que** l'isolement des acides nucléiques a lieu en présence de sels chaotropiques.

12. Procédé selon la revendication 11, **caractérisé en ce que** la concentration en sels chaotropiques est de 2-8 moles/l, de préférence de 4-6 moles/l.

13. Procédé selon la revendication 11 ou 12, **caractérisé en ce que** les sels chaotropiques sont choisis parmi l'iodure de sodium, le perchlorate de sodium, le thiocyanate de guanidinium, l'isothiocyanate de guanidinium et le chlorhydrate de guanidinium.

14. Procédé selon l'une quelconque des revendications 1 à 13, **caractérisé en ce que** l'échantillon est mélangé avec les particules magnétiques et incubé pendant un temps suffisant pour la liaison, de préférence entre 10 secondes et 30 minutes.

15. Procédé selon la revendication 14, **caractérisé en ce qu'**une séparation des acides nucléiques du liquide a lieu après l'incubation à l'aide d'un champ magnétique.

16. Procédé selon l'une quelconque des revendications 1 à 15, **caractérisé en ce que** les particules magnétiques sont purifiées une ou plusieurs fois avec une solution de lavage.

17. Procédé selon la revendication 16, **caractérisé en ce qu'**une étape de séchage a lieu après la dernière étape de lavage, le cas échéant avec un prétraitement à l'acétone.

18. Procédé selon la revendication 16 ou 17, **caractérisé en ce que** les acides nucléiques purifiés sont élués des particules magnétiques avec un tampon d'élution présentant une teneur en sel inférieure à 0,2 mole/l.

19. Procédé selon la revendication 18, **caractérisé en ce que** le tampon d'élution contient du Tris ou est de l'eau déminéralisée.

20. Procédé selon l'une quelconque des revendications 1 à 19, **caractérisé en ce qu'**on réalise comme réaction enzymatique un séquençage, un marquage radioactif ou non radioactif, une amplification d'une ou de plusieurs séquences, une transcription, une hybridation avec des sondes d'acides nucléiques marquées, une traduction ou une ligation.
